# EUROPÄISCHE PATENTSCHRIFT

(11) **EP 1 392 679 B1**
(45) Veröffentlichungstag und Bekanntmachung des Hinweises auf die Patenterteilung: **02.11.2005**
(21) Anmeldenummer: 02754561.5
(22) Anmeldetag: 04.05.2002
(51) Int. Cl.: C07D 401/06, C07D 405/14, A61K 31/454, A61P 1/04, A61P 1/06

(54) **NEUE 1-AMIDOMETHYLCARBONYL-PIPERIDINDERIVATE, VERFAHREN ZU IHRER HERSTELLUNG UND DIESE VERBINDUNGEN ENTHALTENDE ARZNEIMITTEL**
NOVEL 1-AMIDOMETHYLCARBONYL-PIPERIDINE DERIVATIVES, METHOD FOR THE PRODUCTION THEREOF AND MEDICAMENT CONTAINING SAID COMPOUNDS
NOUVEAUX DERIVES DE 1-AMIDOMETHYLCARBONYL-PIPERIDINE, PROCEDE POUR PRODUIRE CES COMPOSES ET MEDICAMENTS CONTENANT CES COMPOSES

(30) Priorität: 10.05.2001 DE 10122603; 21.05.2001 US 291935 P
(43) Veröffentlichungstag der Anmeldung: 03.03.2004
(73) Patentinhaber: Solvay Pharmaceuticals GmbH, 30173 Hannover (DE)
(72) Erfinder: JASSERAND, Daniel, D-30625 Hannover (DE); ANTEL, Jochen, 31848 Bad Münder (DE); PREUSCHOFF, Ulf, 30916 Isernhagen N.B. (DE); BRÜCKNER, Reinhard, 30559 Hannover (DE); SANN, Holger, 30629 Hannover (DE); WURL, Michael, 30826 Garbsen (DE); EICKELMANN, Peter, 88441 Mittelbiberach (DE)
(74) Vertreter: Bauriegel, Lutz
(86) Internationale Anmeldenummer: PCT/EP2002/004904
(87) Internationale Veröffentlichungsnummer: WO 2002/092592

(56) Entgegenhaltungen:
- EP-A- 0 076 530
- WO-A-00/74679
- WO-A-96/39384
- WO-A-99/09991
- GB-A- 2 351 733
- JACOBSON, ALAN R. ET AL: "Minimum-structure enkephalin analogs incorporating L-tyrosine, D (or L)-phenylalanine, and a diamine spacer" J. MED. CHEM. (1989), 32(8), 1708-17 , XP002209707

## Beschreibung

Die vorliegende Erfindung betrifft neue, 4-substituierte 1-Amidomethylcarbonyl-Piperidinderivate mit Motilin-agonistischen Eigenschaften und deren Säureadditionssalze sowie diese Verbindungen enthaltende pharmazeutische Zubereitungen und Verfahren und Zwischenprodukte zur Herstellung dieser Verbindungen.

Motilin-agonistisch wirksame Verbindungen mit macrolidischen Strukturen sind bereits bekannt, beispielsweise aus dem europäischen Patent EP 0 550 895 B1.

Aus der internationalen Patentanmeldung WO 97/38665 sind bereits u. a. 1,4-substituierte Piperidinderivate bekannt, welche als Inhibitoren der Farnesyl-Protein Transferase wirksam sind.

Die internationale Patentanmeldung WO 96/39384 schlägt substituierte N-(Indol-2-carbonyl)-glycinamide als Glycogenphosphorylase Inhibitoren zur Behandlung von u.a. Diabetes, Hyperglykämie und Bluthochdruck vor.

Aus der internationalen Patentanmeldung WO 00/74679 sind bereits substituierte Piperidine als Melanocortin-4-Rezeptorantagonisten bekannt, welche sich zur Behandlung von u.a. Obesitas, Diabetes oder sexueller Störungen eignen.

In der europäischen Patentanmeldung EP 0 076 530 werden N-(3-Hydroxy-4-piperidinyl)benzamid-Derivate offenbart, welche die Motilität des Gastrointestinalsystems stimulieren können.

Die britische Patentanmeldung GB 2351 733 beschreibt schließlich nicht-peptidische Somatostatin-Agonisten, welche sich zur Behandlung von u.a. Diabetes oder Krebs eignen.

Der vorliegenden Erfindung liegt die Aufgabe zugrunde, neue Verbindungen mit günstiger Wirkung auf die Motilität des gastrointestinalen Traktes, insbesondere mit Motilin-artiger Wirkung, zu entwickeln, welche keine von Erythromycin abgeleitete makrocyclische Grundstruktur aufweisen.

Es wurde nun überraschend gefunden, daß die neuen 1-Amidomethylcarbonyl-Piperidinderivate selektive Motilinagonistische Eigenschaften aufweisen und somit geeignet erscheinen, die Motilität des gastrointestinalen Traktes in günstiger Weise zu stimulieren und den Tonus des unteren Ösophagus Sphincter zu verstärken. Aufgrund ihres Wirkungsprofiles erscheinen die erfindungsgemäßen Verbindungen daher geeignet zur Behandlung von Motilitätsstörungen im gastrointestinalen Trakt.

Gegenstand der Erfindung ist daher die Verwendung von 1-Amidomethylcarbonyl-Piperidinderivate in der allgemeinen Formel I 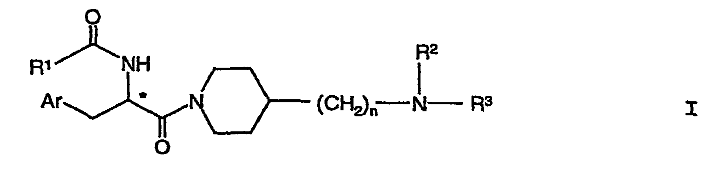 worin
- R¹: Phenyl-C₀₋₄-Alkyl, welches im Phenylring gegebenenfalls durch Niederalkylendioxy oder 1- bis 3-fach durch Halogen, Trifluormethyl, Niederalkyl oder Niederalkoxy substituiert ist,
Heteroaryl-C₀₋₄-alkyl, welches im Heteroarylring gegebenenfalls durch Halogen, Niederalkyl oder Niederalkoxy substituiert ist,
C₁₋₆-Alkyl, welches gegebenenfalls durch Carboxy, Hydroxy, Oxo, Hydroximino, Niederalkyloximino, Amino, Niederalkylamino, Diniederalkylamino oder Niederalkoxy substituiert ist, oder
C₃₋₆-Cycloalkyl
bedeutet,
- R²: C₁₋₈-Alkyl,
Naphthylniederalkyl,
Fluorenylniederalkyl oder
Phenyl-C₀₋₄-alkyl, welches im Phenylring gegebenenfalls substituiert ist durch Niederalkylendioxy oder 1- bis 3-fach durch Trifluormethyl,
Niederalkyl,
Diniederalkylamino,
Niederalkoxy oder
Phenyl-C₀₋₄-alkoxy, welches im Phenylring gegebenenfalls durch Niederalkyl, Niederalkoxy und/oder Niederalkylendioxy substituiert ist,
bedeutet,
- R³: Wasserstoff,
Niederalkyl,
Naphthylniederalkyl,
Fluorenylniederalkyl oder
Phenyl-C₀₋₄-alkyl, welches im Phenylring gegebenenfalls substituiert ist durch
Niederalkylendioxy oder 1- bis 2-fach durch Niederalkyl,
Diniederalkylamino,
Niederalkoxy oder
Phenyl-C₀₋₄-alkoxy, welches im Phenylring gegebenenfalls durch Niederalkyl oder Niederalkoxy substituiert ist,
bedeutet,
- Ar: Phenyl, welches gegebenenfalls durch Niederalkylendioxy oder 1- bis 3-fach durch Halogen, Niederalkyl oder Niederalkoxy substituiert ist,
Naphthyl, welches gegebenenfalls durch Niederalkylendioxy oder 1- bis 3-fach durch Halogen, Niederalkyl oder Niederalkoxy substituiert ist, oder
Indolyl, welches gegebenenfalls durch Halogen, Niederalkyl oder Niederalkoxy substituiert ist,
bedeutet und
- n: eine ganze Zahl zwischen 0 und 3 bedeutet,
sowie deren physiologisch verträglicher Säureadditionssalze zur Herstellung pharmazeutischer Zubereitungen für die Behandlung und/oder Prophylaxe von Krankheitszuständen, welche mit Motilitätsstörungen im gastrointestinalen Trakt und/oder Rückfluss von Speisebrei aus dem Magen in die Speiseröhre verbunden sind.

Gegenstand der Erfindung sind auch neue Verbindungen der Formel Id wie in Anspruch 2 definiert.

Sofern in den Verbindungen der Formel I die Substituenten niedere Alkylgruppen bedeuten oder enthalten, können diese geradkettig oder verzweigt sein und 1 bis 4 Kohlenstoffatome enthalten. Sofern die Substituenten Halogen enthalten, kommen insbesondere Fluor, Chlor oder Brom, vorzugsweise Fluor oder Chlor in Frage. Sofern in Verbindungen der Formel I Substituenten durch Niederalkylendioxy substituiertes Phenyl enthalten, kommen als Niederalkylendioxy insbesondere an zwei benachbarte Atome des Phenylringes gebundene Sauerstoffatome in Frage, welche über eine Niederalkylenkette mit 1 bis 4, insbesondere 1 bis 2 Kohlenstoffatomen verbrückt sind. Vorzugsweise steht Niederalkylendioxy für Methylendioxy.

R¹ steht insbesondere für Phenyl-C₀₋₄-Alkyl. In einer bevorzugten Variante steht R¹ für Phenylethyl. Sofern R¹ für Heteroaxyl-C₀₋₄-alkyl steht, kommen insbesondere elektronenreiche Heteroarylreste in Frage, beispielsweise Furan, Benzofuran, Thiophen, Benzothiophen, Pyrrol oder Indol. Sofern R¹ für C₁₋₆-Alkyl steht, ist dieses vorzugsweise durch mindestens eine der hierfür vorstehend genannten funktionellen Gruppen substituiert.

R² steht vorzugsweise für Fluorenylniederalkyl, insbesondere 2-Fluorenylmethyl, oder für Phenyl-C₀₋₄-Alkyl, welches vorzugsweise substituiert ist. In einer bevorzugten Variante steht R² für in 3,4-Stellung des Phenylringes durch Methylendioxy substituiertes Phenyl-C₀₋₄-Alkyl. In einer anderen bevorzugten Variante steht R² für Phenylmethyl, welches im Phenylring durch Niederalkoxy, insbesondere Methoxy und durch gegebenenfalls durch Niederalkyl substituiertes Phenyl-C₀₋₄-Alkoxy, insbesondere Benzyloxy, substituiert ist.

R³ steht insbesondere für Wasserstoff oder Niederalkyl. Wasserstoff ist bevorzugt.

Ar steht vorzugsweise für gegebenenfalls substituiertes Indolyl. Unsubstituiertes Indolyl ist-bevorzugt. Sofern Ar für gegebenenfalls substituiertes Phenyl steht, ist 3,4-Dichlorphenyl bevorzugt.

Die Verbindungen der Formel I sowie deren Säureadditionssalze können hergestellt werden, indem man
a) zur Herstellung einer Verbindung der allgemeinen Formel Ia 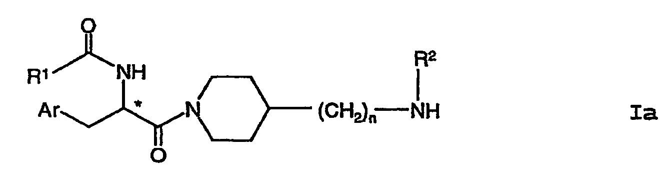 worin R¹, R², Ar und n obige Bedeutungen besitzen, eine Verbindung der Formel II, 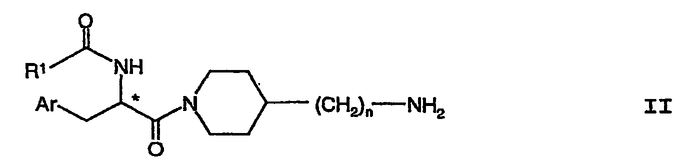 worin R¹, Ar und n obige Bedeutungen besitzen und worin gegebenenfalls vorhandene, unter den Reaktionsbedingungen reaktive funktionelle Gruppen mit geeigneten Schutzgruppen blockiert sind, mit einer Verbindung der allgemeinen Formel III, 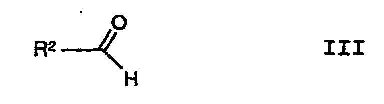 worin R² die obige Bedeutung besitzt und worin gegebenenfalls vorhandene, unter den Reaktionsbedingungen reaktive funktionelle Gruppen mit geeigneten Schutzgruppen blockiert sind, unter Bedingungen einer reduktiven Aminierung umsetzt und nicht gewünschte Schutzgruppen nachfolgend wieder abspaltet, oder
b) zur Herstellung von Verbindungen der allgemeinen Formel Ib 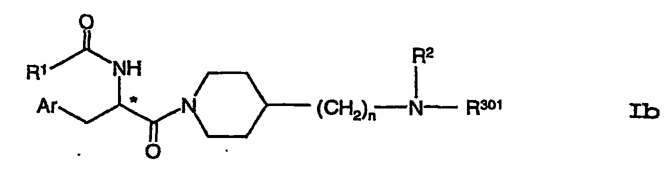 worin R¹, R², Ar und n obige Bedeutungen besitzen und R³⁰¹ die oben für R³ angegebene Bedeutung mit Ausnahme von Wasserstoff besitzt, eine Verbindung der Formel Ia, worin gegebenenfalls vorhandene, unter den Reaktionsbedingungen reaktive funktionelle Gruppen mit geeigneten Schutzgruppen blockiert sind, mit einer Verbindung der allgemeinen Formel IV 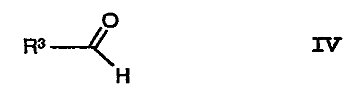 worin R³ die oben angegebene Bedeutung besitzt und worin gegebenenfalls vorhandene, unter den Reaktionsbedingungen reaktive funktionelle Gruppen mit geeigneten Schutzgruppen blockiert sind, unter Bedingungen einer reduktiven Aminierung umsetzt und nicht gewünschte Schutzgruppen nachfolgend wieder abspaltet,
und freie Verbindungen der Formel I gewünschtenfalls in ihre Säureadditionssalze überführt oder Säureadditionssalze der Verbindungen der Formel I in freie Verbindungen überführt.

Gemäß Verfahrensvariante a) kann ein primäres Amin der Formel II mit einem Aldehyd der Formel III in Anwesenheit eines geeigneten Reduktionsmittels auf an sich bekannte Weise unter Bedingungen einer reduktiven Aminierung umgesetzt werden, um eine Verbindung der Formel Ia zu erhalten. Die Umsetzung kann in einem unter den Reaktionsbedingungen inerten Lösungsmittel, vorzugsweise bei Temperaturen zwischen -20 °C und Raumtemperatur erfolgen. Als Lösungsmittel eignen sich beispielsweise halogenierte Kohlenwasserstoffe wie Dichlormethan oder cyclische Ether wie Tetrahydrofuran (THF) oder Dioxan oder Gemische dieser Lösungsmittel. Als Reduktionsmittel eignen sich komplexe Alkalimetallborhydride, insbesondere Natriumtriacetoxyborhydrid. Schutzgruppen, welche sich zur selektiven Einführung und späteren selektiven Abspaltung in der vorstehend angegebenen Verfahrensvariante a) und in den nachfolgend angegebenen Herstellungsverfahren eignen, sind an sich bekannt, beispielsweise aus J. A. W. McOmie, "Protective Groups in Organic Chemistry", Plenum Press oder T. W. Green und P. G. M. Wuts, "Protective Groups in Organic Synthesis", Wiley and Sons, 1991. Der Fachmann kann jeweils geeignete Schutzgruppen durch Routinemethoden auswählen.

Gemäß Verfahrensvariante b) kann ein sekundäres Amin der Formel Ia mit einem Aldehyd der Formel IV in Anwesenheit eines geeigneten Reduktionsmittels auf an sich bekannte Weise unter Bedingungen einer reduktiven Aminierung umgesetzt werden, um eine Verbindung der Formel Ib zu erhalten. Die Umsetzung kann beispielsweise in der vorstehend für die Umsetzung von Verbindungen der Formel II mit Verbindungen der Formel III beschriebenen Weise erfolgen.

Verbindungen der Formel II und deren stereoisomere Formen sind Verbindungen, welche sich als Zwischenprodukte zur Herstellung neuer pharmakologisch aktiver Wirkstoffe, beispielweise zur Herstellung der Verbindungen der Formel I eignen. Die Verbindungen der Formel II können hergestellt werden, indem man Verbindungen der allgemeinen Formel V, 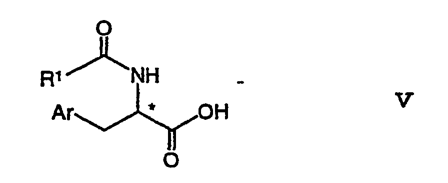 worin R¹ und Ar obige Bedeutungen besitzen, wobei gegebenenfalls vorhandene, unter den Reaktionsbedingungen reaktive funktionelle Gruppen jeweils mit geeigneten Schutzgruppen blockiert sind, mit Verbindungen der allgemeinen Formel VIa, 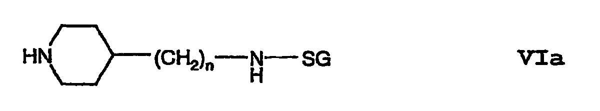 worin n die obige Bedeutung besitzt und SG für eine unter den Reaktionsbedingungen stabile Aminoschutzgruppe steht, umsetzt und nicht gewünschte Schutzgruppen nachfolgend wieder abspaltet. Die Umsetzung der Säuren der Formel V mit den Piperidinderivaten der Formel VIa zu den Amiden der Formel II kann nach an sich zur Bildung von Amidgruppen durch Aminoacylierung üblichen Methoden ausgeführt werden. Als Acylierungsmittel können die Säuren der Formel V oder deren reaktive Derivate wie Säurehalogenide oder gemischte Anhydride der Säuren der Formel V mit Sulfonsäuren wie Toluolsulfonate der Säuren der Formel V eingesetzt werden. Die Acylierung kann in einem unter den Reaktionsbedingungen inerten organischen Lösungsmittel, vorzugsweise bei Temperaturen zwischen -20 °C und Raumtemperatur erfolgen. Als Lösungsmittel eignen sich insbesondere halogenierte Kohlenwasserstoffe wie Dichlormethan oder cyclische Ether wie THF oder Dioxan oder Gemische dieser Lösungsmittel. Die Acylierung kann zweckmäßig in Gegenwart eines säurebindenden Mittels durchgeführt werden. Als säurebindende Mittel eignen sich in dem Reaktionsgemisch lösliche Basen, insbesondere organische Basen wie tertiäre Niederalkylamine und Pyridine, beispielsweise Triethylamin, Tripropylamin oder 4-Dimethylaminopyridin. Falls als Acylierungsmittel die Säuren der Formel V selbst eingesetzt werden, kann die Acylierung zweckmäßig auch in Gegenwart von aus der Peptidchemie als zur Amidbildung geeignet bekannten Kupplungsreagenzien wie Alkylcarbodiimiden, z. B. Dicyclohexylcarbodiimid oder Carbonyldiimidazol, durchgeführt werden.

Verbindungen der Formel V können hergestellt werden, indem man Verbindungen der allgemeinen Formel VII, 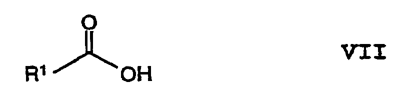 worin R¹ obige Bedeutung besitzt und worin gegebenenfalls vorhandene, unter den Reaktionsbedingungen reaktive funktionelle Gruppen jeweils mit geeigneten Schutzgruppen blockiert sind, mit Verbindungen der allgemeinen Formel VIII, 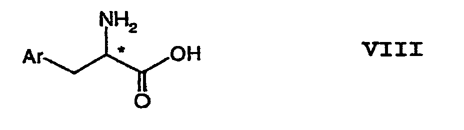 worin Ar obige Bedeutung besitzt, umsetzt. Die Umsetzung kann nach an sich zur Bildung von Amidgruppen durch Aminoacylierung üblichen Methoden, beispielsweise nach der vorstehend für die Umsetzung von Verbindungen der Formel V mit Verbindungen der Formel VIa angegebenen Weise durchgeführt werden.

Die Säuren der Formel VII sind bekannt oder können vom Fachmann durch chemische Routinemethoden aus bekannten Verbindungen hergestellt werden.

Die α-Aminocarbonsäuren der Formel VIII sind bekannt, oder können vom Fachmann durch chemische Routinemethoden aus bekannten Verbindungen hergestellt werden.

Die Verbindungen der Formel VIa können durch Einführung geeigneter Schutzgruppen SG aus den Verbindungen der allgemeinen Formel VI, 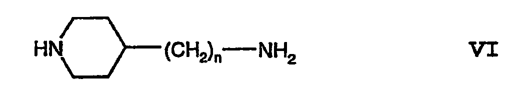 worin n obige Bedeutung besitzt, hergestellt werden. Als Schutzgruppen SG eignen sich insbesondere aus der Peptidchemie bekannte Schutzgruppen, vorzugsweise die tert.-Butyloxycarbonyl-Schutzgruppe (= ^{t}BOC).

Die Verbindungen der Formel VI sind bekannt oder können vom Fachmann durch chemische Routinemethoden aus bekannten Verbindungen hergestellt werden.

Die Verbindungen der Formel I können auf an sich bekannte Weise aus dem Reaktionsgemisch isoliert und gereinigt werden. Säureadditionssalze können in üblicher Weise in die freien Basen überführt werden und diese können gewünschtenfalls in bekannter Weise in physiologisch verträgliche Säureadditionssalze überführt werden.

Als physiologisch verträgliche Salze von Verbindungen der Formel I kommen deren Salze mit anorganischen Säuren, beispielsweise Schwefelsäure, Phosphorsäuren oder Halogenwasserstoffsäuren, vorzugsweise Chlorwasserstoffsäure, oder mit organischen Säuren, beispielsweise niederen aliphatischen Mono-, Di- oder Tricarbonsäuren wie Maleinsäure, Fumarsäure, Milchsäure, Weinsäure, Zitronensäure oder mit Sulfonsäuren, beispielsweise Niederalkansulfonsäuren wie Methansulfonsäure oder gegebenenfalls im Benzolring durch Halogen öder niederes Alkyl substituierte Benzolsulfonsäuren wie p-Toluolsulfonsäure in Frage.

Die Verbindungen der Formel I enthalten jedenfalls ein chirales Kohlenstoffatom, nämlich das mit "*" bezeichnete Kohlenstoffatom in β-Stellung zum Ringstickstoffatom des Piperidinringes. Die Verbindungen der-Formel I können somit in mehreren stereoisomeren Formen vorliegen. Die vorliegende Erfindung erfaßt sowohl die Gemische optischer Isomere als auch die isomerenreinen Verbindungen der Formel I. Bevorzugt sind Verbindungen der Formel I, worin das vorgenannten Chiralitätszentrum die R-Konfiguration aufweist. Falls bei der Synthese der Verbindungen der Formel I Gemische optischer Isomere der Ausgangsverbindungen, beispielsweise der Verbindungen der Formel II oder der Verbindungen der Formel VII, eingesetzt werden, werden auch die Verbindungen der Formel I in Form von Gemischen optischer Isomere erhalten. Ausgehend von stereochemisch einheitlichen Formen der Ausgangsverbindung können auch stereochemisch einheitliche Verbindungen der Formel I erhalten werden. Die stereochemisch einheitlichen Verbindungen der Formel I können aus den Gemischen optischer Isomere in an sich bekannter Weise erhalten werden, z. B. durch chromatographische Trennung an chiralen Trennmaterialien oder durch Umsetzung mit geeigneten optisch aktiven Säuren, beispielsweise Weinsäure oder Campher-10-sulfonsäure, und anschließender Auftrennung in die optisch aktiven Antipoden durch fraktionierte Kristallisation der gewonnenen diastereomeren Salze.

Die neuen Verbindungen der Formel I und ihre physiologisch verträglichen Säureadditionssalze besitzen interessante pharmakologische Eigenschaften, insbesondere die Motilität des gastrointestinalen Traktes stimulierende Motilin-agonistische Eigenschaften.

Um eine geregelte Verdauung der eingenommenen Nahrung zu gewährleisten, wirken im gesunden Zustand das autonome Nervensystem und Hormone des gastrointestinalen Traktes zusammen, um eine geregelte Kontraktionstätigkeit des gastrointestinalen Traktes nicht nur direkt nach Nahrungsaufnahme, sondern auch bei leerem gastrointestinalem Trakt zu erzeugen. Motilin ist ein bekanntes gastrointestinales Peptidhormon, welches die Motilität des gastrointestinalen Traktes stimuliert und eine koordinierende Motilität im gesamten gastrointestinalen Trakt im nüchternen zustand sowie nach Nahrungsaufnahme induziert.

Die Verbindungen der Formel I zeigen motilinartige Wirkungen, indem sie als Agonisten an Motilinrezeptoren wirksam werden. So ist zu erwarten, daß die Verbindungen der Formel I ausgeprägte stimulierende Wirkungen im Magen-Darm-Bereich und am unteren Speiseröhrensphincter zeigen oder auch in anderen Bereichen des Körpers, welche Motilinrezeptoren enthalten, beispielsweise im ZNS. Weiterhin ist zu erwarten, daß die Verbindungen der Formel I eine Beschleunigung der Magenentleerung bewirken. Aufgrund ihrer motilinartigen Wirkungsweise erscheinen die Substanzen geeignet zur Behandlung von Krankheitszuständen, welche mit Motilitätsstörungen im gastrointestinalen Trakt und/oder Rückfluß von Speisebrei aus dem Magen in die Speiseröhre verbunden sind. So sind die Verbindungen der Formel I z. B. induziert bei Gastroparesis verschiedensten Ursprungs, beispielsweise diabetischer Gastroparese, Störungen der Magenentleerung und gastroösophagalem Rückfluß, Dyspepsie, Anomalien der Colonmotilität wie sie z. B. beim Reizdarmsyndrom (= irritable bowel syndrome, IBS) auftreten und postoperativen Motilitätsstörungen, z. B. Darmverschluß (= Ileus) oder auch bei Störungen der Gallenblasenentleerung.

Die gastrointestinal wirksamen Eigenschaften der Verbindungen der Formel I lassen sich in pharmakologischen Standardtestmethoden nachweisen.

### Beschreibung der pharmakologischen Testmethoden

Die Motilin-agonistischen Wirkungen der Testsubstanzen können beispielsweise in einem pharmakologischen Standarcitest in vitro nachgewiesen werden.

Hierfür werden CHO-Zellen (= Ovarzellen des chinesischen Hamsters, "chinese hamster ovary cells") auf an sich bekannte Weise mit je einem Expressionsvektor für den humanen Motilinrezeptor GPR38 (vgl. K. K. McGee et al., Genomics 46 (1997) 426 - 434) sowie für Apoaequorin (vgl. EP 0 187 519 B1 bzw. US 5,162,227; US 5,422,266; US 5,744,579; US 5,766,941 und US 5,798,441) transfiziert.

Die cDNA für den Motilin-Rezeptor (GPR38) wurde aus cDNA aus humanem Magengewebe durch Polymerasekettenreaktion (PCR) mit geeigneten Oligonukleotiden als Primern gewonnen. Die Klonierung des PCR-Produktes erfolgte zunächst in den Klonierungsvektor pCR-blunt (Fa. Invitrogen). Die Bestätigung der klonierten Sequenz erfolgte durch DNA-Sequenzierung. Anschließend wurde die cDNA mit Hilfe der Restriktionsendonukleasen HindIII und SpeI in geeignete Restriktionsschnittstellen (HindIII, Xba I) des Expressionsvektors pcDNA3 (Fa. Invitrogen, NL) subkloniert. Die cDNA für mitochondrial exprimiertes Aequorin wurde aus dem Plasmid mtAEQ/pMT2 (Fa. Molecular Probes, Katalognummer A-6788) durch Restriktionsenzyme herausgeschnitten und in den Expressionsvektor pIRES-puro (Fa. Clontech, Katalognummer 6031-1) subkloniert. Auf das Vorhandensein der beiden Expressionskonstrukte in den transfizierten CHO-Zellen wurde mit G418 und Puromycin selektioniert. Die Zellkultur erfolgte in DMEM/F12 (1:1) Medium (vgl. Life Technologies Kat. Nr. 11039-02, Zusammensetzung der Medien beschrieben in: Dulbecco et al. Virology 8 (1959) 396; Smith et al. Virology 12 (1960) 185; Tissue Culture Standards Committee, In Vitro 6:2, 93. Ham (1965) Proc. Natl. Acad. Sci., 53, 288) mit 10 % (V/V) fetalem Kälberserum, 100 IU/ml Penicillin, 100 µg/ml Streptomycin, 5 µg/ml Puromycin und 400 µg/ml G418.

Der Motilinrezeptor ist über G-Proteine an den Ca²⁺-Signaltransduktionsweg der CHO-Zelle gekoppelt, d. h. eine Aktivierung des Rezeptors führt zu einer transienten Erhöhung der intrazellulären Ca²⁺-Konzentration. Das durch Beladung der Apoaequorin exprimierenden CHO-Zellen mit Coelenterazin entstandene Aequorin kann mit den mitochondrialen Ca²⁺-Ionen der CHO-Zellen unter Freisetzung von Chemilumineszenz reagieren. Die freigesetzte Chemilumineszenz ist proportional zur Konzentration der eingesetzten Motilin-agonistisch wirksamen Verbindungen der Formel I. Im vorliegenden Testmodell werden die Verbindungen der Formel I in gleichbleibenden Konzentrationen gemessen. Die gemessene Chemilumineszenz ist daher ein Maß für die Wirksamkeit der Testverbindungen als Motilin-Agonisten.

Der pharmakologische Test wird nach einem aus der WO 00/02045 bekannten Verfahren durchgeführt.

Mit dem GPR38-Rezeptor transfizierte CHO-Zellen in ihrer logarithmischen Wachstumsphase wurden von ihren Kulturschalen mit Phosphat-gepufferter, Ca²⁺-Ionen-freier Kochsalzlösung, welche einen Zusatz von 5 mM Ethylendiamintetraacetat enthielt (= PBS-EDTA-Lösung) abgelöst und abzentrifugiert, der Überstand wurde verworfen. Die verbliebenen Pellets wurden in einem BSA-Medium [= DMEM/F12(1:1)-Medium mit HEPES (15 M), 0,1 % Rinderserumalbumin (bovine serum albumine, BSA), 100 IU/ml Penicillin, 100 µg/ml Streptomycin, ohne Phenolrot] resuspendiert. Anschließend wurden die Zellen in einer Neubauer-Kammer gezählt, erneut zentrifugiert und in dem vorgenannten BSA-Medium resuspendiert, so daß die Zelldichte 5 X 10⁶-Zellen/ml betrug. Hierzu wurde von einer 500 µM Stammlösung von Coelenterazin h in Methanol soviel hinzugefügt, daß eine Endkonzentration von 5 µM in dem BSA-Medium erreicht wurde. Die Zellen wurden in einem mit Aluminiumfolie umwickelten Becherglas auf einem Magnetrührer durch langsames Rühren in Suspension gehalten und insgesamt 4 Stunden lang bei Raumtemperatur inkubiert, um aktives Aequorin zu erhalten. Anschließend wurden die Zellen noch einmal mit dem vorgenannten BSA-Medium auf das 10-fache Volumen verdünnt und 30 Minuten lang bei Raumtemperatur gerührt. Lösungen eines Referenzagonisten (als Referenzagonist wurde Motilin in einer Konzentration von 10⁻⁷ M verwendet) und Testverbindungen (1 % in DMSO) wurden in den Löchern einer Probenplatte mit 96 Probenplätzen ("96-well plate") vorgelegt (jeweils 10 µl). Zu dieser Vorlage wurden jeweils 90 µl der vorstehend zubereiteten zellsuspension (i. e. 45.000 Zellen) über den Injektor eines Microbeta-Jet (Fa. Wallac) zupipettiert und das emittierte Licht wurde über 15 Sekunden gemessen und integriert. Auf diese Weise erhielt man für jeden Probenplatz einen repräsentativen Wert für das emittierte Licht und demzufolge für die Stärke der Stimulation des Motilin-Rezeptors durch die in dem entsprechenden Probenplatz vorhandene Verbindung der Formel I.

Für die Verbindungen der untenstehenden Beispiele wurde die Stärke der Stimulation des humanen Motilin-Rezeptors GPR38 jeweils durch einmalige Messung der Testsubstanzen in einer Konzentration von 10⁻⁵ M bestimmt. Alle Testsubstanzen der Beispiele 1 bis 16 zeigten in diesem Testmodell eine Stimulation des humanen GPR38-Motilinrezeptors, die mindestens 40 % der Stimulation dieses Rezeptors durch den Referenzagonisten Motilin betrug. Die Verbindungen der Beispiele 6 bis 16 zeigten eine Stimulation von jeweils mindestens 60 %, bezogen auf die Stimulation durch den Referenzagonisten Motilin. Die angegebenen Beispielsnummern beziehen sich auf die nachfolgenden Herstellungsbeispiele.

Die Verbindungen der Formel I können in üblichen pharmazeutischen Zubereitungen verabreicht werden. Die zu verwendenden Dosen können individuell verschieden sein und variieren naturgemäß je nach Art des zu behandelnden Zustandes und der verwendeten Substanz. Im allgemeinen eignen sich zur Applikation am Menschen und an größeren Säugetieren jedoch Arzneiformen mit einem Wirkstoffgehalt von 0,1 bis 800 mg, insbesondere 1 bis 100 mg Wirkstoff pro Einzeldosis.

Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt oder auch Suppositorien. Diese Präparate können pharmazeutisch übliche anorganische und/oder organische Trägerstoffe, wie z. B. Talkum, Milchzucker oder Stärke, neben pharmazeutisch üblichen Hilfsmitteln, beispielsweise Gleitmitteln oder Tablettensprengmitteln, enthalten. Flüssige Präparate wie Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfs-und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die nachfolgend angeführten Beispiele sollen die Erfindung näher erläutern, ohne sie in ihrem Umfang zu beschränken.

### Beispiel 1:

### N-[(1RS)-2-({[3-(1,3-benzodioxol-5-yl)-((2RS)-methylpropyl]amino}-1-piperidinyl)-1-(1H-indol-3-ylmethyl)-2-oxo-ethyl]-3-phenylpropanamid

A) 15,0 g NaOH wurden in 150 ml Wasser gelöst und es wurden 51,0 g DL-Tryptophan zugegeben. Anschließend tropfte man zu dieser Vorlage unter Rühren und Eiskühlung eine Lösung von 37,0 ml 3-Phenylpropionsäurechlorid in 75 ml THF zu. Nachdem weitere 45 Minuten lang bei Raumtemperatur gerührt worden war, wurde der pH-Wert in der Reaktionslösung durch tropfenweise Zugabe von konzentrierter HCl auf 3 - 4 eingestellt. Der nach Zugabe von wenig Wasser erhaltene weiße Feststoff wurde durch Vakuumfiltration abgetrennt. Man erhielt 59,8 g DL-N-Phenylpropionyltryptophan vom Schmelzpunkt (= Fp.) 175 bis 176 °C.
B) 3,4 g des vorstehend erhaltenen Tryptophanderivates wurden bei Raumtemperatur und unter Schutzgasatmosphäre in 200 ml THF gelöst, mit 1,7 g Carbonyldiimidazol versetzt und 1 Stunde lang bei Raumtemperatur gerührt. Zu dieser Vorlage tropfte man anschließend eine Lösung von 2,0 g 4-(tert.-Butyloxycarbonylamino)piperidin in 25 ml Dichlormethan hinzu, ließ noch eine Stunde lang bei Raumtemperatur weiterrühren und anschließend ließ man das Reaktionsgemisch über Nacht stehen. Dann dampfte man überschüssiges THF im Vakuum ein und nahm den Rückstand in 200 ml Essigsäureethylester auf. Die organische Phase wurde der Reihe nach mit 2 mal 50 ml Wasser, 1 mal mit 15 ml 15%iger wäßriger Weinsäurelösung, 3 mal mit 50 ml Wasser, 1 mal mit 50 ml verdünnter wäßriger Kaliumcarbonatlösung und schließlich 2 mal mit 50 ml Wasser gewaschen. Anschließend wurde die organische Phase über Natriumsulfat getrocknet und zur Trockene eingedampft. Man erhielt 4,5 g N-[3-phenylpropionyl]-DL-tryptophan-(4-(tert.-butoxycarbonyl)amino)piperidinamid als weißen Feststoff, Fp = 105 - 110 °C
C) Aus mehreren Ansätzen der vorstehend unter B) beschriebenen Reaktion wurde das erhaltene Produkt zu insgesamt 25,0 g vereinigt und unter Schutzgasatmosphäre in 800 ml Methanol suspendiert. Zu dieser Vorlage gab man 90 ml einer 37%igen wäßrigen HCl unter Rühren zu und ließ über Nacht stehen. Anschließend dampfte man überschüssiges Methanol weitgehend ein, nahm den Rückstand in 100 ml Wasser auf und wusch die wäßrige Phase mit wenig Essigsäureethylester. Die wäßrige Phase wurde abgetrennt und durch Zugabe von verdünnter wäßriger Natronlauge auf einen pH-Wert von 8 gebracht. Man extrahierte die wäßrige Phase mit ca. 100 ml Essigsäureethylester, filtrierte vom ausgefallenen Niederschlag ab und wusch die organische Phase mit gesättigter wäßriger Kochsalzlösung. Anschließend trocknete man die organische Phase und dampfte das Lösungsmittel bis.zur Trockene ein. Man erhielt 13,5 g N-[3-Phenylpropionyl]-DL-tryptophan(4-amino)-piperidinamid als weißes Pulver, welches nach Chromatographie (Kieselgel, Laufmittel: erst Dichlormethan, dann Dichlormethan/Ethanol/Triethylamin 90:7:3) und Umkristallisierung aus n-Pentan einen Fp. von 70 - 73 °C aufwies.
D) 400 mg des vorstehend unter C) erhaltenen Produktes wurden in 40 ml 1,2-Dichlorethan bei Raumtemperatur und unter Schutzgasatmosphäre gelöst. Zu dieser Vorlage wurden 0,19 ml 3-(3,4-Methylendioxyphenyl)-2-methylpropan-aldehyd und nach einigen Minuten dann 550 mg Natriumtriacetoxyborhydrid zugegeben. Es wurde 60 Stunden lang bei Raumtemperatur gerührt und anschließend mit 30 ml gesättigter wäßriger Natriumhydrogencarbonatlösung gewaschen. Man extrahierte die wäßrige Phase mit 30 ml Dichlormethan und trocknete die vereinigten organischen Phasen über Natriumsulfat. Man dampfte bis fast zur Trockene ein und reinigte den verbleibenden Rückstand durch Säulenchromatographie (Kieselgel, Laufmittel: erst Essigester, welchem nach und nach Ethanol bis zu einem Verhältnis 9:1 zugemischt wurde). Vereinigung und Trocknung der Produktphasen lieferte 590 mg der Titelverbindung als weißlichen Feststoff.
E) 360 mg der vorstehend erhaltenen Produkt-Base wurden bei 50 °C in 15 ml Methyl-tert.-butylether gelöst. Zu dieser Vorlage gab man 0,5 ml isopropanolischer HCl zu und ließ noch 10 Minuten lang bei ca. 50 °C rühren. Man ließ das Reaktionsgemisch über Nacht bei Raumtemperatur stehen, filtrierte den ausgefallenen Feststoff ab und trocknete diesen bei 60 °C im Vakuum. Man erhielt 350 mg eines Hydrochlorids der Titelverbindung, Fp. = 124 - 127 °C.

### Beispiel 2:

### N-[(1R)-2-(4{[3-(1,3-benzodioxol-5-yl)-(2RS)-methylpropyl]amino}-1-piperidinyl)-1-(1H-indol-3-ylmethyl)-2-oxo-ethyl]-3-phenylpropanamid

A) 30,28 g D-(-)-Tryptophan wurden mit 28,38 ml 3-Phenylpropionsäurechlorid auf die in Beispiel 1A) angegebene Weise umgesetzt. Man erhielt 16,88 g D-N-Phenylpropionyltryptophan als weißes Pulver.
B) 5,13 g des vorstehend erhaltenen D-(-)-Tryptophanderivates wurden mit 3,0 g 4-(tert.-Butoxycarbonylamino)piperidin auf die in Beispiel 1B) angegebene Weise umgesetzt. Man erhielt 6,5 g N-[3-Phenylpropionyl-D-tryptophan-(4-(tert.-butoxycarbonyl)amino)piperidinamid als weißen Feststoff, Fp. = 105 - 110 °C.
C) 6,2 g des vorstehend erhaltenen Produktes wurden durch Zugabe von 37%iger wäßriger HCl auf die in Beispiel 1C) angegebene Weise entschützt. Man erhielt 2,3 g N-[3-Phenylpropionyl]-D-tryptophan-(4-amino)-piperidinamid als weißlichen Schaum.
D) 719 mg des vorstehend erhaltenen Produktes wurden mit 0,33 ml 3-(3,4-Methylendioxyphenyl)-2-methylpropanaldehyd auf die in Beispiel 1D) angegebene Weise umgesetzt. Man erhielt 670 mg der Titelverbindung als Schaum.
E) 630 mg der vorstehend erhaltenen Titelverbindung wurden auf die in Beispiel 1E) angegebene Weise in das Hydrochlorid überführt. Man erhielt 640 mg eines Hydrochlorids der Titelverbindung vom Fp. = 140° - 145 °C; optischer Drehwert [α]_{D}²⁰ = 9,3° (c = 1,0 in Methanol).

Nach den vorstehend angegebenen Herstellungsverfahren oder analog zu diesen Herstellungsverfahren können auch die nachfolgend angegebenen Verbindungen der Beispiele 3 bis 16 hergestellt werden.

Die nachfolgenden Verbindungen der Beispiele 3 bis 16 wurden nach einem automatisierten Herstellungsverfahren hergestellt. Hierzu wurden Vorratsreagenzien von Natriumtriacetoxyborhydrid (0,25 M Suspension in Chloroform) und dem als Reaktionspartner jeweils vorgesehenen Aldehyd der Formel III (0,25 M in Chloroform) vorbereitet. Daraufhin wurden in einem Mikroreaktionsgefäß jeweils 200 µl racemisches N-[3-Phenylpropionyl]tryptophan-(4-amino)-piperidinamid der Formel Ia (0,25 M in Chloroform), 500 µl des Natriumacetoxyborhydrid-Vorratsreagenzes und 200 µl des Aldehyd-Vorratsreagenzes zusammengegeben. Das Reaktionsgefäß wurde verschlossen und das Reaktionsgemisch wurde über Nacht geschüttelt. Die erhaltenen Rohprodukte von Verbindungen der Formel I wurden jeweils mit 1,2 ml Chloroform verdünnt und mit 2,0 ml einer 1 N wäßrigen NaOH extrahiert. Die überstehenden Phasen wurden verworfen und die unteren Phasen wurden jeweils mit 2,0 ml Wasser gewaschen. Die nunmehr entstandenen unteren Phasen wurden erneut in Mikroreaktionsgefäße überführt und die überstehenden Phasen wurden je einmal mit Chloroform extrahiert. Die vereinigten organischen Phasen wurden jeweils bis fast zur Trockene eingedampft und die erhaltenen Rückstände wurden jeweils in 1,8 ml Dimethylsulfoxid (= DMSO) aufgenommen. Aus den erhaltenen DMSO-Lösungen wurde ohne weitere Aufreinigung jeweils eine Probe für die Hochleistungs-Flüssigkeitschromatographie (= HPLC) bzw. für die automatische Massenspektroskopie zur Bestimmung der Reinheit bzw. zur Strukturbestätigung entnommen.

Alle in der nachfolgenden Tabelle 1 angegebenen Verbindungen der Formel I weisen an dem mit "*" gekennzeichneten Chiralitätszentrum in β-Stellung zu dem Ringstickstoffatom des Piperidinringes racemische Konfiguration auf.

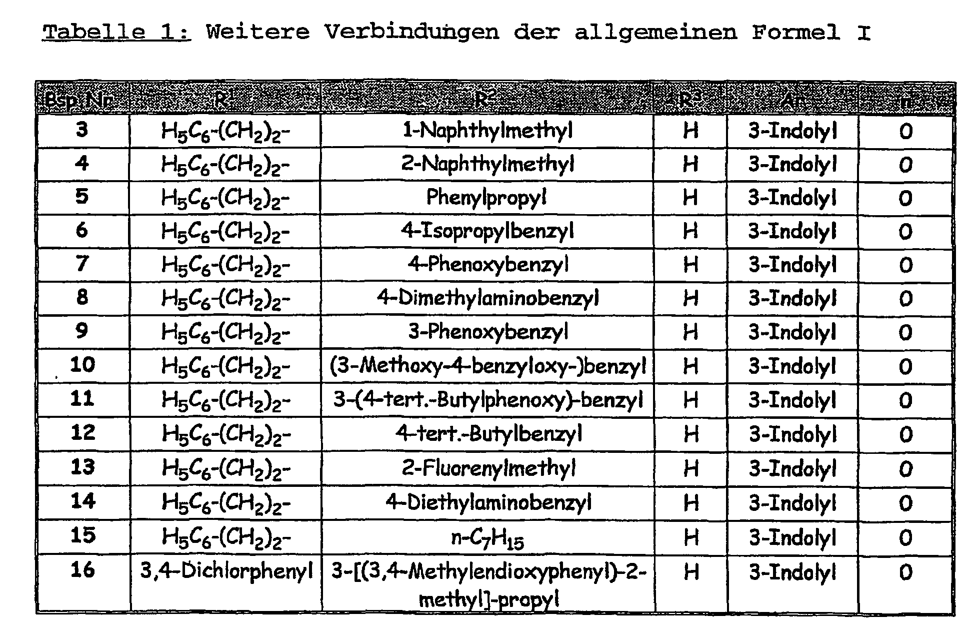

### Beispiel I:

### N-[(1R)-2-(4{[3-(1,3-benzodioxol-5-yl)-(2RS)-methylpropyl]amino}-1-piperidinyl)-1-(1H-indol-3-ylmethyl)-2-oxo-ethyl]-3-phenylpropanamid enthaltende Kapseln:

Es werden Kapseln in folgender Zusammensetzung pro Kapsel hergestellt:

| | |
|---|---|
| *N*-[(1*R*)-2-(4{[3-(1,3-benzodioxol-5-yl)-(2*RS*)-methylpropyl]amino}-1-piperidinyl)-1-(1*H*-indol-3-ylmethyl)-2-oxoethyl]-3-phenylpropanamid | 20 mg |
| Maisstärke | 60 mg |
| Milchzucker | 300 mg |
| Ethylacetat | q.s. |

Der Wirkstoff, die Maisstärke und der Milchzucker werden unter Zuhilfenahme von Ethylacetat zu einer homogenen pastösen Mischung verarbeitet. Die Paste wird zerkleinert und das entstehende Granulat wird auf ein geeignetes Blech gebracht und zur Entfernung des Lösungsmittels bei 45 °C getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann in 400 mg fassende Kapseln (= Kapselgröße 0) abgefüllt.

## Patentansprüche

1. Verwendung von Verbindungen der allgemeinen Formel I 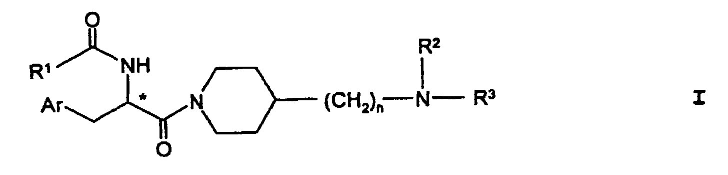 worin
R¹ Phenyl-C₀₋₄-alkyl, welches im Phenylring gegebenenfalls durch C₁₋₄-Alkylendioxy oder 1- bis 3-fach durch Halogen, Trifluormethyl, C₁₋₄-Alkyl oder C₁₋₄-Alkoxy substituiert ist,
Heteroaryl-C₀₋₄-alkyl, welches im Heteroarylring gegebenenfalls durch Halogen, C₁₋₄-Alkyl oder C₁₋₄-Alkoxy substituiert ist,
C₁₋₆-Alkyl, welches gegebenenfalls durch Carboxy, Hydroxy, Oxo, Hydroximino, C₁₋₄-Alkyloximino, Amino, C₁₋₄-Alkylamino, Di-C₁₋₄-alkylamino oder C₁₋₄-Alkoxy substituiert ist, oder
C₃₋₆-Cycloalkyl
bedeutet,
R² C₁₋₈-Alkyl, Naphthyl-C₁₋₄-alkyl,
Fluorenyl-C₁₋₄-alkyl oder Phenyl-C₀₋₄-alkyl, welches im Phenylring gegebenenfalls substituiert ist durch
C₁₋₄-Alkylendioxy oder 1- bis 3-fach durch
Trifluormethyl,
C₁₋₄-Alkyl,
Di-C₁₋₄-alkylamino,
C₁₋₄-Alkoxy oder
Phenyl-C₀₋₄-alkoxy, welches im Phenylring gegebenenfalls durch C₁₋₄-Alkyl, C₁₋₄-Alkoxy und/oder C₁₋₄-Alkylendioxy substituiert ist,
bedeutet,
R³ Wasserstoff,
C₁₋₄-Alkyl,
Naphthyl-C₁₋₄-alkyl,
Fluorenyl-C₁₋₄-alkyl oder
Phenyl-C₀₋₄-alkyl, welches im Phenylring gegebenenfalls substituiert ist durch
C₁₋₄-Alkylendioxy oder 1- bis 2-fach durch
C₁₋₄-Alkyl,
Di-C₁₋₄-Alkylamino,
C₁₋₄-Alkoxy oder
Phenyl-C₀₋₄-alkoxy, welches im Phenylring gegebenenfalls durch C₁₋₄-Alkyl oder C₁₋₄-Alkoxy substituiert ist,
bedeutet,
Ar Phenyl, welches gegebenenfalls durch C₁₋₄-Alkylendioxy oder 1- bis 3-fach durch Halogen, C₁₋₄-Alkyl oder C₁₋ ₄-Alkoxy substituiert ist,
Naphthyl, welches gegebenenfalls durch C₁₋₄-Alkylendioxy oder 1- bis 3-fach durch Halogen, C₁₋₄-Alkyl oder C₁₋₄-Alkoxy substituiert ist, oder
Indolyl, welches gegebenenfalls durch Halogen, C₁₋₄-Alkyl oder C₁₋₄-Alkoxy substituiert ist,
bedeutet und
n eine ganze Zahl zwischen 0 und 3 bedeutet,
sowie deren physiologisch verträglicher Säureadditionssalze zur Herstellung pharmazeutischer Zubereitungen für die Behandlung und/oder Prophylaxe von Krankheitszuständen, welche mit Motilitätsstörungen im gastrointestinalen Trakt und/oder Rückfluß von Speisebrei aus dem Magen in die Speiseröhre verbunden sind.

2. Verbindungen der allgemeinen Formel Id 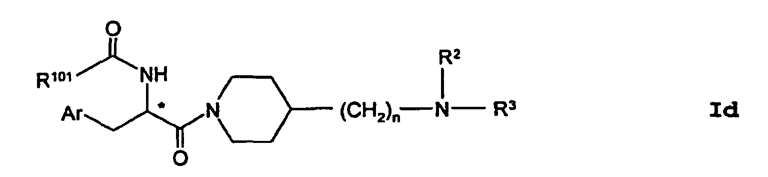 worin
R¹⁰¹ Phenyl-C₀₋₄-alkyl, welches im Phenylring gegebenenfalls durch C₁₋₄-Alkylendioxy oder 1- bis 3-fach durch Halogen, Trifluormethyl, C₁₋₄-Alkyl oder C₁₋₄-Alkoxy substituiert ist,
Furan, Benzofuran, Thiophen, Benzothiophen oder Pyrrol, welches im Heteroarylring gegebenenfalls jeweils durch Halogen, C₁₋₄-Alkyl oder C₁₋₄-Alkoxy substituiert ist,
C₁₋₆-Alkyl, welches gegebenenfalls durch Carboxy, Hydroxy, Oxo, Hydroximino, C₁₋₄-Alkyloximino, Amino, C₁₋₄-Alkylamino, Di-C₁₋₄-alkylamino oder C₁₋₄-Alkoxy substituiert ist, oder
C₃₋₆-Cycloalkyl
bedeutet,
R² C₁₋₈-Alkyl, Naphthyl-C₁₋₄-alkyl,
Fluorenyl-C₁₋₄-alkyl oder Phenyl-C₀₋₄-alkyl, welches im Phenylring gegebenenfalls substituiert ist durch
C₁₋₄-Alkylendioxy oder 1- bis 3-fach durch Trifluormethyl,
C₁₋₄-Alkyl,
Di-C₁₋₄-alkylamino,
C₁₋₄-Alkoxy oder
Phenyl-C₀₋₄-alkoxy, welches im Phenylring gegebenenfalls durch C₁₋₄-Alkyl, C₁₋₄-Alkoxy und/oder C₁₋₄ -Alkylendioxy substituiert ist,
bedeutet,
R³ Wasserstoff,
C₁₋₄-Alkyl,
Naphthyl-C₁₋₄-alkyl,
Fluorenyl-C₁₋₄-alkyl oder
Phenyl-C₀₋₄-alkyl, welches im Phenylring gegebenenfalls substituiert ist durch
C₁₋₄-Alkylendioxy oder 1- bis 2-fach durch
C₁₋₄-Alkyl,
Di-C₁₋₄-alkylamino,
C₁₋₄-Alkoxy oder
Phenyl-C₀₋₄-alkoxy, welches im Phenylring gegebenenfalls durch C₁₋₄-Alkyl oder C₁₋₄-Alkoxy substituiert ist,
bedeutet,
Ar Phenyl, welches gegebenenfalls durch C₁₋₄-Alkylendioxy oder 1- bis 3-fach durch Halogen, C₁₋₄-Alkyl oder C₁₋₄ -Alkoxy substituiert ist,
Naphthyl, welches gegebenenfalls durch C₁₋₄-Alkylendioxy oder 1- bis 3-fach durch Halogen, C₁₋₄-Alkyl oder C₁₋₄-Alkoxy substituiert ist, oder
Indolyl, welches gegebenenfalls durch Halogen, C₁₋₄-Alkyl oder C₁₋₄-Alkoxy substituiert ist,
bedeutet und
n eine ganze Zahl zwischen 0 und 3 bedeutet,
sowie deren physiologisch verträgliche Säureadditionssalze.

3. Verbindungen der allgemeinen Formel Id nach Anspruch 2, worin Ar gegebenenfalls substituiertes Indolyl bedeutet.

4. Verbindungen der allgemeinen Formel Id nach Anspruch 2, worin R³ Wasserstoff bedeutet.

5. Verbindungen der allgemeinen Formel Id nach Anspruch 2, worin das Chiralitätszentrum C* die R-Konfiguration besitzt.

6. Arzneimittel, enthaltend eine pharmakologisch wirksame Menge einer Verbindung der allgemeinen Formel Id nach Anspruch 2 sowie übliche Hilfs- und/oder Trägerstoffe.

7. Verfahren zur Herstellung von Verbindungen der allgemeinen Formel Id 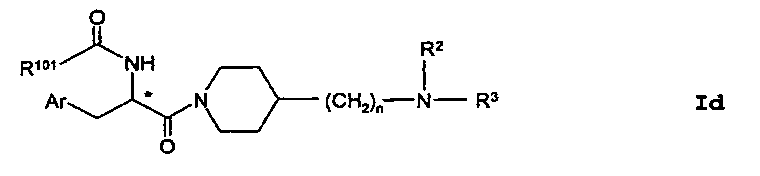 worin
R¹⁰¹ Phenyl-C₀₋₄-alkyl, welches im Phenylring gegebenenfalls durch C₁₋₄-Alkylendioxy oder 1- bis 3-fach durch Halogen, Trifluormethyl, C₁₋₄-Alkyl oder C₁₋₄-Alkoxy substituiert ist,
Furan, Benzofuran, Thiophen, Benzothiophen oder Pyrrol, welches im Heteroarylring gegebenenfalls jeweils durch Halogen, C₁₋₄-Alkyl oder C₁₋₄-Alkoxy substituiert ist,
C₁₋₆-Alkyl, welches gegebenenfalls durch Carboxy, Hydroxy, Oxo, Hydroximino, C₁₋₄-Alkyloximino, Amino, C₁₋₄-Alkylamino, Di-C₁₋₄-alkylamino oder C₁₋₄-Alkoxy substituiert ist, oder
C₃₋₆-Cycloalkyl
bedeutet,
R² C₁₋₈-Alkyl, Naphthyl-C₁₋₄-alkyl,
Fluorenyl-C₁₋₄-alkyl oder
Phenyl-C₀₋₄-alkyl, welches im Phenylring gegebenenfalls substituiert ist durch
C₁₋₄-Alkylendioxy oder 1- bis 3-fach durch
Trifluormethyl,
C₁₋₄-Alkyl,
Di-C₁₋₄-alkylamino,
C₁₋₄-Alkoxy oder
Phenyl-C₀₋₄-alkoxy, welches im Phenylring gegebenenfalls durch C₁₋₄-Alkyl, C₁₋₄-Alkoxy und/oder C₁₋₄ -Alkylendioxy substituiert ist,
bedeutet,
R³ Wasserstoff,
C₁₋₄-Alkyl,
Naphthyl-C₁₋₄-alkyl,
Fluorenyl-C₁₋₄-alkyl oder
Phenyl-C₀₋₄-alkyl, welches im Phenylring gegebenenfalls substituiert ist durch
C₁₋₄-Alkylendioxy oder 1- bis 2-fach durch
C₁₋₄-Alkyl,
Di-C₁₋₄-alkylamino,
C₁₋₄-Alkoxy oder
Phenyl-C₀₋₄-alkoxy, welches im Phenylring gegebenenfalls durch C₁₋₄-Alkyl oder C₁₋₄-Alkoxy substituiert ist,
bedeutet,
Ar Phenyl, welches gegebenenfalls durch C₁₋₄-Alkylendioxy oder 1- bis 3-fach durch Halogen, C₁₋₄-Alkyl oder C₁₋₄ -Alkoxy substituiert ist,
Naphthyl, welches gegebenenfalls durch C₁₋₄-Alkylendioxy oder 1- bis 3-fach durch Halogen, C₁₋₄-Alkyl oder C₁₋₄-Alkoxy substituiert ist, oder
Indolyl, welches gegebenenfalls durch Halogen, C₁₋₄-Alkyl oder C₁₋₄-Alkoxy substituiert ist,
bedeutet und
n eine ganze Zahl zwischen 0 und 3 bedeutet,
sowie deren stabilen und physiologisch verträglichen Säureadditionssalzen, **dadurch gekennzeichnet, dass** man
a) zur Herstellung einer Verbindung der allgemeinen Formel Ia' 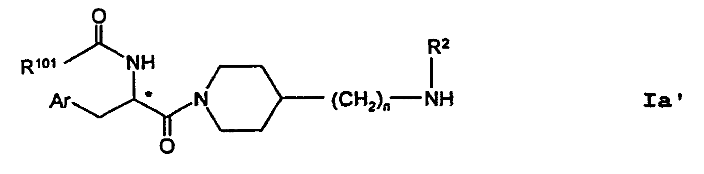 worin R¹⁰¹, R², Ar und n obige Bedeutungen besitzen, eine Verbindung der Formel II', 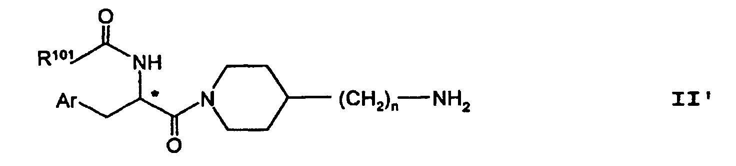 worin R¹⁰¹, Ar und n obige Bedeutungen besitzen und worin gegebenenfalls vorhandene, unter den Reaktionsbedingungen reaktive funktionelle Gruppen mit geeigneten Schutzgruppen blockiert sind, mit einer Verbindung der allgemeinen Formel III, 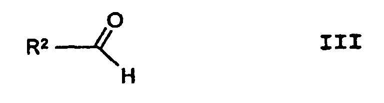 worin R² die obige Bedeutung besitzt und worin gegebenenfalls vorhandene, unter den Reaktionsbedingungen reaktive funktionelle Gruppen mit geeigneten Schutzgruppen blokkiert sind, unter Bedingungen einer reduktiven Aminierung umsetzt und nicht gewünschte Schutzgruppen nachfolgend wieder abspaltet, oder
b) zur Herstellung von Verbindungen der allgemeinen Formel Ib' 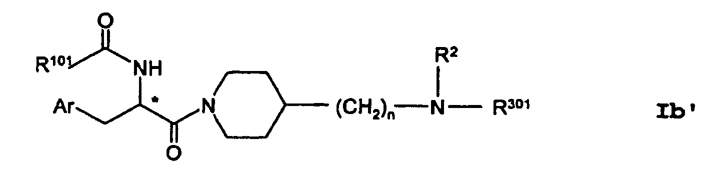
worin R¹⁰¹, R², Ar und n obige Bedeutungen besitzen und R³⁰¹ die oben für R³ angegebene Bedeutung mit Ausnahme von Wasserstoff besitzt, eine Verbindung der Formel Ia', worin gegebenenfalls vorhandene, unter den Reaktionsbedingungen reaktive funktionelle Gruppen mit geeigneten Schutzgruppen blockiert sind, mit einer Verbindung der allgemeinen Formel IV 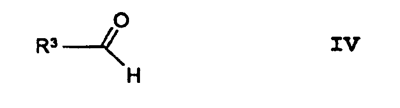 worin R³ die oben angegebene Bedeutung besitzt und worin gegebenenfalls vorhandene, unter den Reaktionsbedingungen reaktive funktionelle Gruppen mit geeigneten Schutzgruppen blockiert sind, unter Bedingungen einer reduktiven Aminierung umsetzt und nicht gewünschte Schutzgruppen nachfolgend wieder abspaltet,
und freie Verbindungen der Formel I gewünschtenfalls in ihre Säureadditionssalze überführt oder Säureadditionssalze der Verbindungen der Formel I in freie Verbindungen überführt.

## Claims

1. The use of compounds of the general formula I 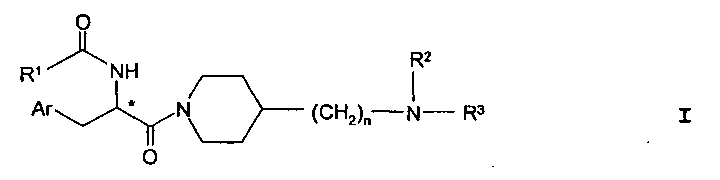 wherein.
R¹ is phenyl-C₀₋₄-alkyl which is optionally substituted in the phenyl ring by C₁₋₄-alkylenedioxy or 1 to 3 times by halogen, trifluoromethyl, C₁₋₄-alkyl or C₁₋₄-alkoxy,
heteroaryl-C₀₋₄-alkyl which is optionally substituted in the heteroaryl ring by halogen, C₁₋₄-alkyl or C₁₋₄-alkoxy,
C₁₋₆-alkyl which is optionally substituted by carboxy, hydroxy, oxo, hydroximino, C₁₋₄-alkyloximino, amino, C₁₋₄-alkylamino, di-C₁₋₄-alkylamino or C₁₋₄-alkoxy, or
C₃₋₆-cycloalkyl,
R² is C₁₋₈-alkyl, naphthyl-C₁₋₄-alkyl, fluorenyl-C₁₋₄-alkyl or
phenyl-C₀₋₄-alkyl which is optionally substituted in the phenyl ring by
C₁₋₄-alkylenedioxy or 1 to 3 times by trifluoromethyl,
C₁₋₄-alkyl,
di-C₁₋₄-alkylamino,
C₁₋₄-alkoxy or
phenyl-C₀₋₄-alkoxy which is optionally substituted in the phenyl ring by C₁₋₄-alkyl, C₁₋₄-alkoxy and/or C₁₋₄ -alkylenedioxy,
R³ is hydrogen,
C₁₋₄-alkyl,
naphthyl-C₁₋₄-alkyl,
fluorenyl-C₁₋₄-alkyl or
phenyl-C₀₋₄-alkyl which is optionally substituted in the phenyl ring by
C₁₋₄-alkylenedioxy or 1 to 2 times by
C₁₋₄-alkyl,
di-C₁₋₄-alkylamino,
C₁₋₄-alkoxy or
phenyl-C₀₋₄-alkoxy which is optionally substituted in the phenyl ring by C₁₋₄-alkyl or C₁₋₄-alkoxy,
Ar is phenyl which is optionally substituted by C₁₋₄-alkylenedioxy or 1 to 3 times by halogen, C₁₋₄-alkyl or C₁₋₄-alkoxy,
naphthyl which is optionally substituted by C₁₋₄-alkylenedioxy or 1 to 3 times by halogen, C₁₋₄-alkyl or C₁₋₄-alkoxy, or
indolyl which is optionally substituted by halogen, C₁₋₄-alkyl or C₁₋₄-alkoxy,
and
n is a whole number between 0 and 3,
and their physiologically compatible acid addition salts for the preparation of pharmaceutical preparations for the treatment and/or prophylaxis of pathological conditions which are associated with motility disturbances in the gastrointestinal tract and/or reflux of chyme from the stomach into the oesophagus.

2. Compounds of the general formula Id 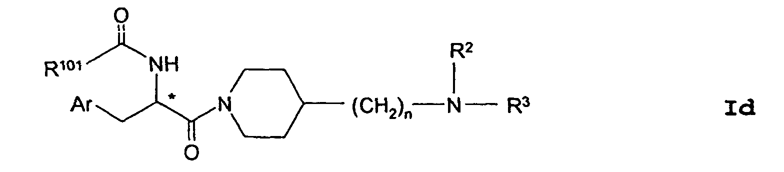 wherein
R¹⁰¹ is phenyl-C₀₋₄-alkyl which is optionally substituted in the phenyl ring by C₁₋₄-alkylenedioxy or 1 to 3 times by halogen, trifluoromethyl, C₁₋₄-alkyl or C₁₋₄-alkoxy,
furan, benzofuran, thiophene, benzothiophene or pyrrole which is substituted in the heteroaryl ring optionally in each case by halogen, C₁₋₄-alkyl or C₁₋₄-alkoxy,
C₁₋₆-alkyl which is optionally substituted by carboxy, hydroxy, oxo, hydroximino, C₁₋₄-alkyloximino, amino, C₁₋₄-alkylamino, di-C₁₋₄-alkylamino or C₁₋₄-alkoxy, or
C₃₋₆-cycloalkyl,
R² is C₁₋₈-alkyl, naphthyl-C₁₋₄-alkyl, fluorenyl-C₁₋₄-alkyl or
phenyl-C₀₋₄-alkyl which is optionally substituted in the phenyl ring by
C₁₋₄-alkylenedioxy or 1 to 3 times by trifluoromethyl,
C₁₋₄-alkyl,
di-C₁₋₄-alkylamino,
C₁₋₄-alkoxy or
phenyl-C₀₋₄-alkoxy which is optionally substituted in the phenyl ring by C₁₋₄-alkyl, C₁₋₄-alkoxy and/or C₁₋₄-alkylenedioxy,
R³ is hydrogen,
C₁₋₄-alkyl,
naphthyl-C₁₋₄-alkyl,
fluorenyl-C₁₋₄-alkyl or
phenyl-C₀₋₄-alkyl which is optionally substituted in the phenyl ring by
C₁₋₄-alkylenedioxy or 1 to 2 times by C₁₋₄-alkyl,
di-C₁₋₄-alkylamino,
C₁₋₄-alkoxy or
phenyl-C₀₋₄-alkoxy which is optionally substituted in the phenyl ring by C₁₋₄-alkyl or C₁₋₄-alkoxy,
Ar is phenyl which is optionally substituted by C₁₋₄-alkylenedioxy or 1 to 3 times by halogen, C₁₋₄-alkyl or C₁₋₄-alkoxy,
naphthyl which is optionally substituted by C₁₋₄-alkylenedioxy or 1 to 3 times by halogen, C₁₋₄-alkyl or C₁₋₄-alkoxy, or
indolyl which is optionally substituted by halogen, C₁₋₄-alkyl or C₁₋₄-alkoxy,
and
n is a whole number between 0 and 3,
and their physiologically compatible acid addition salts.

3. Compounds of the general formula Id according to Claim 2, wherein Ar is optionally substituted indolyl.

4. Compounds of the general formula Id according to Claim 2, wherein R³ is hydrogen.

5. Compounds of the general formula Id according to Claim 2, wherein the chiral centre C* is in the R configuration.

6. Medicament, containing a pharmacologically active quantity of a compound of the general formula Id according to Claim 2 and conventional auxiliaries and/or carriers.

7. A process for the preparation of compounds of the general formula Id 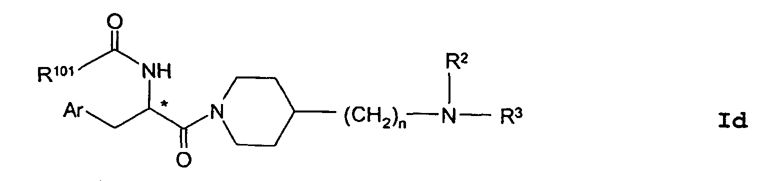 wherein
R¹⁰¹ is phenyl-C₀₋₄-alkyl which is optionally substituted in the phenyl ring by C₁₋₄-alkylenedioxy or 1 to 3 times by halogen, trifluoromethyl, C₁₋₄-alkyl or C₁₋₄-alkoxy,
furan, benzofuran, thiophene, benzothiophene or pyrrole which is substituted in the heteroaryl ring optionally in each case by halogen, C₁₋₄-alkyl or C₁₋₄-alkoxy,
C₁₋₆-alkyl which is optionally substituted by carboxy, hydroxy, oxo, hydroximino, C₁₋₄-alkyloximino, amino, C₁₋₄-alkylamino, di-C₁₋₄-alkylamino or C₁₋₄-alkoxy, or
C₃₋₆-cycloalkyl,
R² is C₁₋₈-alkyl, naphthyl-C₁₋₄-alkyl, fluorenyl-C₁₋₄-alkyl or
phenyl-C₀₋₄-alkyl which is optionally substituted in the phenyl ring by
C₁₋₄-alkylenedioxy or 1 to 3 times by trifluoromethyl,
C₁₋₄-alkyl,
di-C₁₋₄-alkylamino,
C₁₋₄-alkoxy or
phenyl-C₀₋₄-alkoxy which is optionally substituted in the phenyl ring by C₁₋₄-alkyl, C₁₋₄-alkoxy and/or C₁₋₄-alkylenedioxy,
R³ is hydrogen,
C₁₋₄-alkyl,
naphthyl-C₁₋₄-alkyl,
fluorenyl-C₁₋₄-alkyl or
phenyl-C₀₋₄-alkyl which is optionally substituted in the phenyl ring by
C₁₋₄-alkylenedioxy or 1 to 2 times by
C₁₋₄-alkyl,
di-C₁₋₄-alkylamino,
C₁₋₄-alkoxy or
phenyl-C₀₋₄-alkoxy which is optionally substituted in the phenyl ring by C₁₋₄-alkyl or C₁₋₄-alkoxy,
Ar is phenyl which is optionally substituted by C₁₋₄-alkylenedioxy or 1 to 3 times by halogen, C₁₋₄-alkyl or C₁₋₄-alkoxy,
naphthyl which is optionally substituted by C₁₋₄-alkylenedioxy or 1 to 3 times by halogen, C₁₋₄-alkyl or C₁₋₄-alkoxy, or
indolyl which is optionally substituted by halogen, C₁₋₄-alkyl or C₁₋₄-alkoxy,
and
n is a whole number between 0 and 3,
and the stable and physiologically compatible acid addition salts thereof, **characterised in that**
a) for the preparation of a compound of the general formula Ia' 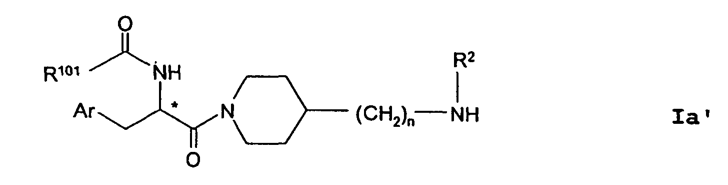 wherein R¹⁰¹, R², Ar and n have the above meanings, a compound of Formula II', 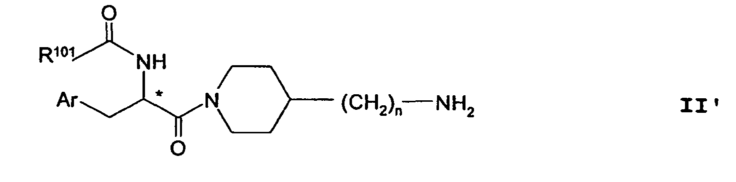 wherein R¹⁰¹, Ar and n have the above meanings and wherein optionally present functional groups which are reactive under the reaction conditions are blocked with suitable protective groups, is reacted with a compound of the general formula III, 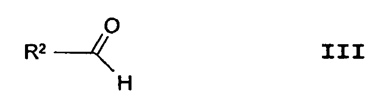 wherein R² has the above meaning and wherein optionally present functional groups which are reactive under the reaction conditions are blocked with suitable protective groups, under conditions of reductive amination and unwanted protective groups are subsequently cleaved off again, or
b) for the preparation of compounds of the general formula Ib' 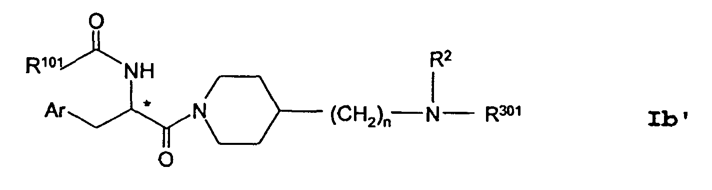 wherein R¹⁰¹, R², Ar and n have the above meanings and R³⁰¹ has the meaning given above for R³ with the exception of hydrogen, a compound of Formula Ia', wherein optionally present functional groups which are reactive under the reaction conditions are blocked with suitable protective groups, is reacted with a compound of the general formula IV 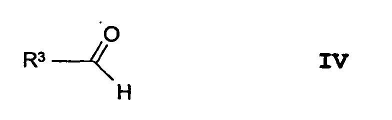 wherein R³ has the above meaning and wherein optionally present functional groups which are reactive under the reaction conditions are blocked with suitable protective groups, under conditions of reductive amination and unwanted protective groups are subsequently cleaved off again,
and free compounds of Formula I if desired are converted into their acid addition salts or acid addition salts of the compounds of Formula I are converted into free compounds.

## Revendications

1. Utilisation de composés de formule générale I : 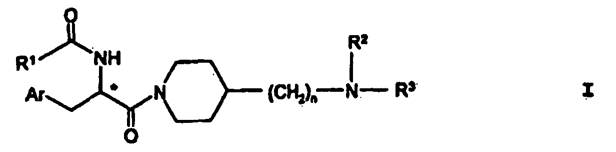 dans laquelle
R¹ représente un groupe alkyl en C₀ à C₄ -phényle, éventuellement substitué dans le noyau phényle par un groupe alkylène en C₁ à C₄ -dioxy, ou une à trois fois par un halogène, un groupe trifluorométhyle, un groupe alkyle en C₁ à C₄ ou un groupe alcoxy en C₁ à C₄,
un groupe alkyl en C₀ à C₄ -hétéroaryle, éventuellement substitué dans le noyau hétéroaryle par un halogène, un groupe alkyle en C₁ à C₄ ou un groupe alcoxy en C₁ à C₄,
un groupe alkyle en C₁ à C₆, éventuellement substitué par un groupe carboxy, hydroxy, oxo, hydroximino, alkyl en C₁ à C₄ -oximino, amino, alkyl en C₁ à C₄ -amino, dialkyl en C₁ à C₄ -amino ou alcoxy en C₁ à C₄, ou
un groupe cycloalkyle en C₃ à C₆,
R² représente un groupe alkyle en C₁ à C₈, alkyl en C₁ à C₄ - naphtyle,
un groupe alkyl en C₁ à C₄ -fluorényle ou
un groupe alkyl en C₀ à C₄ -phényle, éventuellement substitué dans le noyau phényle par
un groupe alkylène en C₁ à C₄ -dioxy, ou une à trois fois par
un groupe trifluorométhyle, alkyle en C₁ à C₄, dialkyl en C₁ à C₄ - amino, alcoxy en C₁ à C₄, ou
un groupe alcoxy en C₀ à C₄ -phényle, éventuellement substitué dans le noyau phényle par un groupe alkyle en C₁ à C₄, un groupe alcoxy en C₁ à C₄ et/ou un groupe alkylène en C₁ à C₄ -dioxy,
R³ représente un atome d'hydrogène,
un groupe alkyle en C₁ à C₄,
un groupe alkyl en C₁ à C₄ -naphtyle,
un groupe alkyl en C₁ à C₄ -fluorényle ou
un groupe alkyl en C₀ à C₄ -phényle, éventuellement substitué dans le noyau phényle par un groupe alkylène en C₁ à C₄ -dioxy, ou une ou deux fois par
un groupe alkyle en C₁ à C₄, dialkyl en C₁ à C₄ -amino, alcoxy en C₁ à C₄, ou
un groupe alcoxy en C₀ à C₄ -phényle, éventuellement substitué dans le noyau phényle par un groupe alkyle en C₁ à C₄ ou un groupe alcoxy en C₁ à C₄,
Ar représente un groupe phényle, éventuellement substitué par un groupe alkylène en C₁ à C₄ -dioxy, ou une à trois fois par un halogène, un groupe alkyle en C₁ à C₄ ou un groupe alcoxy en C₁ à C₄,
un groupe naphtyle, éventuellement substitué par un groupe alkylène en C₁ à C₄ -dioxy, ou une à trois fois par un halogène, un groupe alkyle en C₁ à C₄ ou un groupe alcoxy en C₁ à C₄, ou
un groupe indolyle, éventuellement substitué par un halogène, un groupe alkyle en C₁ à C₄ ou un groupe alcoxy en C₁ à C₄, et
n est un nombre entier compris entre 0 et 3,
ainsi que leurs sels d'addition d'acides physiologiquement compatibles, pour l'élaboration de préparations pharmaceutiques destinées au traitement et/ou à la prophylaxie d'états pathologiques associés à des troubles de la motilité dans le tractus gastro-intestinal et/ou aux reflux vers l'oesophage du chyme provenant de l'estomac.

2. Composés de formule générale Id : 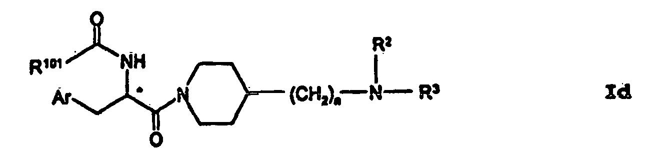 dans laquelle
R¹⁰¹ représente un groupe alkyl en C₀ à C₄ -phényle, éventuellement substitué dans le noyau phényle par un groupe alkylène en C₁ à C₄ -dioxy, ou une à trois fois par un halogène, un groupe trifluorométhyle, un groupe alkyle en C₁ à C₄ ou un groupe alcoxy en C₁ à C₄,
un groupe furane, benzofurane, thiophène, benzothiophène ou pyrrole,
éventuellement substitué dans le noyau hétéroaryle respectivement par un halogène, un groupe alkyle en C₁ à C₄ ou un groupe alcoxy en C₁ à C₄,
un groupe alkyle en C₁ à C₆, éventuellement substitué par un groupe carboxy, hydroxy, oxo, hydroximino, alkyl en C₁ à C₄ -oximino, amino, alkyl en C₁ à C₄ -amino, dialkyl en C₁ à C₄ -amino ou alcoxy en C₁ à C₄, ou
un groupe cycloalkyle en C₃ à C₆,
R² représente un groupe alkyle en C₁ à C₈, alkyl en C₁ à C₄ - naphtyle,
un groupe alkyl en C₁ à C₄ -fluorényle ou
un groupe alkyl en C₀ à C₄ -phényle, éventuellement substitué dans le noyau phényle par
un groupe alkylène en C₁ à C₄ -dioxy, ou une à trois fois par
un groupe trifluorométhyle, alkyle en C₁ à C₄, dialkyl en C₁ à C₄ -amino, alcoxy en C₁ à C₄, ou
un groupe alcoxy en C₀ à C₄ -phényle, éventuellement substitué dans le noyau phényle par un groupe alkyle en C₁ à C₄, un groupe alcoxy en C₁ à C₄ et/ou un groupe alkylène en C₁ à C₄ -dioxy,
R³ représente un atome d'hydrogène,
un groupe alkyle en C₁ à C₄,
un groupe alkyl en C₁ à C₄ -naphtyle,
un groupe alkyl en C₁ à C₄ -fluorényle ou
un groupe alkyl en C₀ à C₄ -phényle, éventuellement substitué dans le noyau phényle par un groupe alkylène en C₁ à C₄ -dioxy, ou une ou deux fois par
un groupe alkyle en C₁ à C₄, dialkyl en C₁ à C₄ -amino, alcoxy en C₁ à C₄, ou
un groupe alcoxy en C₀ à C₄ -phényle, éventuellement substitué dans le noyau phényle par un groupe alkyle en C₁ à C₄ ou un groupe alcoxy en C₁ à C₄,
Ar représente un groupe phényle, éventuellement substitué par un groupe alkylène en C₁ à C₄ -dioxy, ou une à trois fois par un halogène, un groupe alkyle en C₁ à C₄ ou un groupe alcoxy en C₁ à C₄,
un groupe naphtyle, éventuellement substitué par un groupe alkylène en C₁ à C₄ -dioxy, ou une à trois fois par un halogène, un groupe alkyle en C₁ à C₄ ou un groupe alcoxy en C₁ à C₄, ou
un groupe indolyle, éventuellement substitué par un halogène, un groupe alkyle en C₁ à C₄ ou un groupe alcoxy en C₁ à C₄, et
n est un nombre entier compris entre 0 et 3,
ainsi que leurs sels d'addition d'acides physiologiquement compatibles.

3. Composés de formule générale Id selon la revendication 2, dans laquelle Ar représente un groupe indolyle éventuellement substitué.

4. Composés de formule générale Id selon la revendication 2, dans laquelle R³ représente un atome d'hydrogène.

5. Composés de formule générale Id selon la revendication 2, où le centre de chiralité C* possède une configuration R.

6. Médicament comprenant une quantité pharmacologiquement efficace d'un composé de formule générale Id selon la revendication 2, ainsi que des auxiliaires et/ou des excipients traditionnels.

7. Procédé de préparation de composés de formule générale Id : 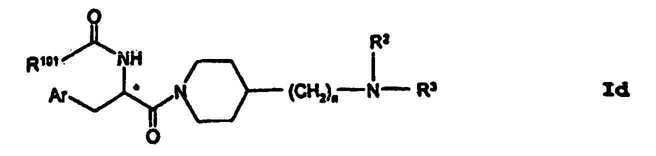 dans laquelle
R¹⁰¹ représente un groupe alkyl en C₀ à C₄ -phényle, éventuellement substitué dans le noyau phényle par un groupe alkylène en C₁ à C₄ -dioxy, ou une à trois fois par un halogène, un groupe trifluorométhyle, un groupe alkyle en C₁ à C₄ ou un groupe alcoxy en C₁ à C₄,
un groupe furane, benzofurane, thiophène, benzothiophène ou pyrrole,
éventuellement substitué dans le noyau hétéroaryle respectivement par un halogène, un groupe alkyle en C₁ à C₄ ou un groupe alcoxy en C₁ à C₄,
un groupe alkyle en C₁ à C₆, éventuellement substitué par un groupe carboxy, hydroxy, oxo, hydroximino, alkyl en C₁ à C₄ -oximino, amino, alkyl en C₁ à C₄ -amino, dialkyl en C₁ à C₄ -amino ou alcoxy en C₁ à C₄, ou
un groupe cycloalkyle en C₃ à C₆,
R² représente un groupe alkyle en C₁ à C₈, alkyl en C₁ à C₄ - naphtyle,
un groupe alkyl en C₁ à C₄ -fluorényle ou
un groupe alkyl en C₀ à C₄ -phényle, éventuellement substitué dans le noyau phényle par
un groupe alkylène en C₁ à C₄ -dioxy, ou une à trois fois par
un groupe trifluorométhyle, alkyle en C₁ à C₄, dialkyl en C₁ à C₄ -amino, alcoxy en C₁ à C₄, ou
un groupe alcoxy en C₀ à C₄ -phényle, éventuellement substitué dans le noyau phényle par un groupe alkyle en C₁ à C₄, un groupe alcoxy en C₁ à C₄ et/ou un groupe alkylène en C₁ à C₄ -dioxy,
R³ représente un atome d'hydrogène,
un groupe alkyle en C₁ à C₄,
un groupe alkyl en C₁ à C₄ -naphtyle,
un groupe alkyl en C₁ à C₄ -fluorényle ou
un groupe alkyl en C₀ à C₄ -phényle, éventuellement substitué dans le noyau phényle par un groupe alkylène en C₁ à C₄ -dioxy, ou une ou deux fois par
un groupe alkyle en C₁ à C₄, dialkyl en C₁ à C₄ -amino, alcoxy en C₁ à C₄, ou
un groupe alcoxy en C₀ à C₄ -phényle, éventuellement substitué dans le noyau phényle par un groupe alkyle en C₁ à C₄ ou un groupe alcoxy en C₁ à C₄,
Ar représente un groupe phényle, éventuellement substitué par un groupe alkylène en C₁ à C₄ -dioxy, ou une à trois fois par un halogène, un groupe alkyle en C₁ à C₄ ou un groupe alcoxy en C₁ à C₄,
un groupe naphtyle, éventuellement substitué par un groupe alkylène en C₁ à C₄ -dioxy, ou une à trois fois par un halogène, un groupe alkyle en C₁ à C₄ ou un groupe alcoxy en C₁ à C₄, ou
un groupe indolyle, éventuellement substitué par un halogène, un groupe alkyle en C₁ à C₄ ou un groupe alcoxy en C₁ à C₄, et
n est un nombre entier compris entre 0 et 3,
ainsi que leurs sels d'addition d'acides stables et physiologiquement compatibles,
**caractérisé en ce que**
a) pour préparer un composé de formule générale Ia' : 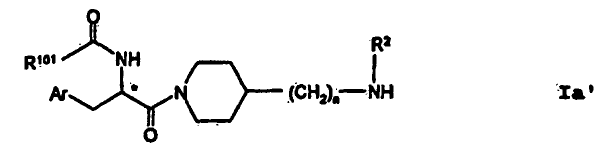 dans laquelle R¹⁰¹, R², Ar et n sont tels que définis ci-avant, l'on fait réagir dans des conditions d'amination réductrice un composé de formule II' : 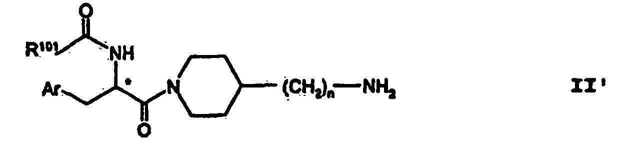 dans laquelle R¹⁰¹, Ar et n sont tels que définis ci-avant et où les groupes fonctionnels, réactifs dans les conditions de la réaction, éventuellement présents sont bloqués par des groupes protecteurs appropriés, avec un composé de formule générale III : 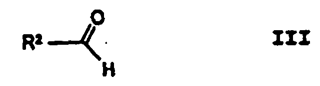 dans laquelle R² est tel que défini ci-avant et où les groupes fonctionnels, réactifs dans les conditions de la réaction, éventuellement présents sont bloqués par des groupes protecteurs appropriés, les groupes protecteurs indésirables étant ensuite de nouveau séparés, ou
b) pour préparer des composés de formule générale Ib' : 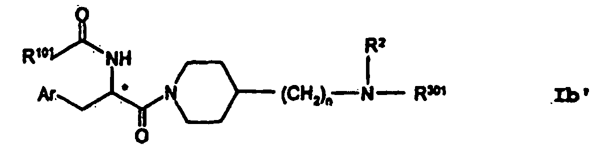
dans laquelle R¹⁰¹, R², Ar et n sont tels que définis ci-avant et R³⁰¹ est tel que R³ est défini ci-avant exception faite de l'atome d'hydrogène, l'on fait réagir dans des conditions d'amination réductrice un composé de formule Ia', où les groupes fonctionnels, réactifs dans les conditions de la réaction, éventuellement présents sont bloqués par des groupes protecteurs appropriés, avec un composé de formule générale IV : 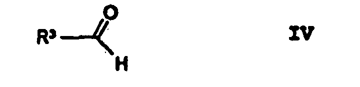 dans laquelle R³ est tel que défini ci-avant et où les groupes fonctionnels, réactifs dans les conditions de la réaction, éventuellement présents sont bloqués par des groupes protecteurs appropriés, les groupes protecteurs indésirables étant ensuite de nouveau séparés,
et l'on convertit au besoin les composés libres de formule I en leurs sels d'addition d'acides ou les sels d'addition d'acides des composés de formule I en composés libres.
